(19) Europäisches Patentamt
European Patent Office
Office européen des brevets

(11) Publication number: **0 503 087 A1**

## EUROPEAN PATENT APPLICATION
### published in accordance with Art. 158(3) EPC

(21) Application number: **91916947.4**

(51) Int. Cl.⁵: **B01D 69/02**, B01D 71/02

(22) Date of filing: **30.09.91**

(86) International application number:
**PCT/JP91/01311**

(87) International publication number:
**WO 92/05865 (16.04.92 92/09)**

(30) Priority: **28.09.90 JP 260744/90**
**28.09.90 JP 260745/90**
**30.10.90 JP 294743/90**
**30.10.90 JP 294744/90**

(43) Date of publication of application:
**16.09.92 Bulletin 92/38**

(84) Designated Contracting States:
**DE FR NL**

(71) Applicant: **TOTO LTD.**
**1-1, Nakajima 2-chome Kokurakita-ku**
**Kitakyushu-shi Fukuoka 802(JP)**

(72) Inventor: **SHIMIZU, Yasutoshi**
**Toto Ltd., 1-1, Nakajima 2-chome,**
**Kokurakita-ku**
**Kitakyushu-shi, Fukuoka 802(JP)**
Inventor: **SHIMODERA, Kenichi**
**Toto Ltd., 1-1, Nakajima 2-chome,**
**Kokurakita-ku**
**Kitakyushu-shi, Fukuoka 802(JP)**

(74) Representative: **Klunker . Schmitt-Nilson .**
**Hirsch**
**Winzererstrasse 106**
**W-8000 München 40(DE)**

(54) **FILTRATION FILM.**

(57) A filtration film capable of collecting with a high efficiency only macromolecular components from a microorganism culture solution containing particle components and macromolecular components. It is characterized in that it has pores whose mean diameter is more than 10 nm and less than 10 $\mu$m or which provide fractionation molecular weight represented by more than $1 \times 10^4$ and less than $1 \times 10^8$ and whose permeation resistance is more than $5 \times 10\ 10^{11}\mathrm{m}^{-1}$ and less than $1 \times 10^{15}\mathrm{m}^{-1}$.

FERMENTED SOLUTION

SOLUTION PASSED THROUGH MEMBRANE ACCORDING TO PRESENT INVENTION

ELUTION TIME ( min )

F I G . I

## TECHNICAL FIELD

The present invention relates to a filtration membrane used for filtration of solution, and more particularly to a filtration membrane which can recover only a macromolecular component with a high efficiency from a culture solution of a microorganism containing a particulate component and the macromolecular component, for example, a biopolymer.

## BACKGROUND ART

In recent years, a separation technique using a membrane has been put to practical use in many fields. For example, the separation of a solvent and an ion from each other by the reverse osmosis method, the separation of a solvent and a solute from each other and the separation of a particulate component in the solvent by microfiltration, etc. have been put to practical use.

Examples of the membrane material used in these separation techniques include organic compounds such as cellulose acetate, polyacrylonitrile, polyamide, polysulfone and polyethylene and inorganic compounds such as aluminum oxide, zirconium oxide and silicon oxide. The membrane made from an organic compound can be produced by a method such as evaporation, coacervation or drawing. On the other hand, the membrane made from an inorganic compound can be produced by a method wherein an inorganic compound particle is sintered, a method wherein use is made of a phase separation phenomenon or the like.

Meanwhile, a macromolecular component, such as protein, is, in general, recovered by filtration from a culture solution obtained by the fermentation of a microorganism. When a protein or the like is produced by fermentation of microorganism and the intended macromolecular substance is recovered from the culture solution, the intended macromolecular substance is recovered by filtration from the cultivation product per se or from a mixture obtained by disrupting bacterial cells.

A method wherein a disrupted cultivation product is allowed to flow parallel to a surface of a porous membrane, i.e., a cross flow filtration, is used as a preferable method of recovering the intended high-molecular substance. A proper selection of the pore diameter of the porous membrane enables an intended macromolecular component to be passed through the membrane with the other bacteria cells or their disrupting products (hereinafter referred to as a "particulate component") being left without passing through the membrane. Thus, only the intended macromolecular component can be isolated.

Since the macromolecular component can pass through a porous membrane together with a solvent, a membrane having a high permeability to a solvent has been considered advantageous as a porous membrane for use in the above-described filtration. In particular, a membrane comprising an organic compound has been regarded as advantageous for the reason that it can have a high porosity, and because a high permeability to a solvent can be imparted. That is, in the case of a membrane comprising an organic compound, it is possible to increase the pure water permeation flux (PWF) which is an amount of permeation of a solvent through the membrane per unit time and unit area. The permeability to a solvent can be evaluated in terms of the permeation resistance, Rm, of the membrane. The Rm can be defined by the following equations (1):

$$Rm = (\Delta P/\eta) \cdot (1/PWF) \qquad (1)$$

wherein $\Delta P$ represents a filtration pressure, $\eta$ represents a viscosity of a solvent and PWF represents a membrane permeation flux value of a solvent.

The permeation resistance, Rm, of the conventional membrane was substantially $1 \times 10^{11}$ m$^{-1}$ or less.

Also in the membrane comprising an inorganic compound, as described in, for example, Japanese Patent Publication Nos. 48646/1983 and 500221/1986, particles having a different particle diameter are piled up to increase the solvent permeability of the membrane (that is, to lower the permeation resistance of the membrane).

An attempt to isolate an intended macromolecular substance by the above-described conventional membrane has unfavorably brought about the following problem. In the case of the conventional membrane, the components (mainly particulate components) which could not permeate through the membrane are accumulated on the surface of the membrane to form a layer. This layer is relatively dense and, in some cases, inhibits the permeation of the intended macromolecular substance to prevent the intended macromolecular substance from efficiently permeating through the membrane.

In order to prevent the formation of the accumulated layer, a method wherein a solution to be filtered is flowed at a high flow rate may be considered to be effective. In this method, however, a large shearing force is applied to the components in the solution, which often unfavorably causes the intended macromolecular substance to be denatured.

For this reason, it is hoped to develop a porous membrane for efficiently recovering a macromolecular component alone from a mixture of an intended macromolecular component with a particulate component.

## SUMMARY OF THE INVENTION

Accordingly, an object of the present invention is to provide a filtration membrane capable of efficiently recovering a macromolecular component alone from a solution containing an intended macromolecular substance and a particulate component.

The filtration membrane according to the present invention is characterized by pores having an average pore diameter in the range from 10 nm to 10 $\mu$m or pores having a cut-off molecular weight in the range from $1 \times 10^4$ to $1 \times 10^8$, said membrane having a permeation resistance in the range from $5 \times 10^{11}$ m$^{-1}$ to $1 \times 10^{15}$ m$^{-1}$.

## BRIEF DESCRIPTION OF THE DRAWINGS

Fig. 1 shows a gel chromatogram for a filtrate by filtering a cultivation product of a microorganism through the filtration membrane according to the present invention, and a gel chromatogram for the cultivation product before the filtration;

Fig. 2 shows a gel chromatogram for a filtrate obtained by filtering a model solution of a disrupted product of bacterial cells through the filtration membrane according to the present invention, and a gel chromatogram for a model solution before the filtration; and

Fig. 3 shows a gel chromatogram for a filtrate by filtering a cultivation product of a microorganism through the conventional filtration membrane, and a gel chromatogram for the cultivation product before the filtration.

## DETAILED DESCRIPTION OF THE INVENTION

### Filtration Membrane

The filtration membrane according to the present invention has pores which have an average pore diameter in the range from 10 nm to 10 $\mu$m, preferably in the range from 10 nm to 1 $\mu$m. Basically, the pore diameter may be set between the size of a component to be permeated in the solution to be filtered and the size of a component not to be permeated. For example, the size of the microorganism is generally in the range from about 1/10 $\mu$m to 10 $\mu$m, and the size of the particulate component in the disrupted cultivation product is generally 10 nm or more. A macromolecular component produced by a microorganism generally has a molecular weight in the range from about $10^3$ to $10^5$ and a size in the range from about several nanometers to several tens of nanometers. Therefore, an intended macromolecular compound produced by a microorganism can be permeated and recovered from a cultivation product or a disrupted cultivation product of a microorganism by means of a filtration membrane with pores having an average pore diameter in the above-described range.

The pores of the filtration membrane may be defined by the cut-off molecular weight. In this case as well, the cut-off molecular weight may be set between the size of a component to be permeated in the solution to be filtered and the size of a component not to be permeated. As described above, the molecular weight of the macromolecular component is in the range from about $10^3$ to $10^5$. Therefore, an intended macromolecular component produced by a microorganism can be permeated and recovered from a cultivation product or a disrupted cultivation product of a microorganism when the filtration membrane has pores having a cut-off molecular weight in the range from $1 \times 10^4$ to $1 \times 10^8$, preferably in the range from $1 \times 10^5$ to $1 \times 10^8$.

Further, the filtration membrane according to the present invention has a permeation resistance in the range from $5 \times 10^{11}$ m$^{-1}$ to $1 \times 10^{15}$ m$^{-1}$, preferably in the range from $6 \times 10^{11}$ m$^{-1}$ to $1 \times 10^{14}$ m$^{-1}$. The permeation resistance of the membrane is defined by the above formula (1). The permeation resistance, therefore, varies from solvent to solvent in the solution, and the value of the permeation resistance is set by taking into consideration the kind of the solvent for the solution to be filtered. For example, when the cultivation product of a microorganism is selected as the solution to be filtered, the membrane is constructed so that the permeation resistance with respect to pure water is in the above range, because the cultivation product is generally in the form of an aqueous solution.

When the permeation resistance is less than $5 \times 10^{11}$ m$^{-1}$, "the accumulated layer" of the particulate component is unfavorably formed on the surface of the membrane. On the other hand, when the permeation resistance exceeds $1 \times 10^{15}$ m$^{-1}$, although the formation of the accumulated layer of the particulate component is not observed, the membrane permeation flux becomes so small that the membrane hardly functions as a filtration membrane.

Although the mechanism through which the formation of the accumulated layer of the particulate component is prevented is not fully elucidated, it is believed that when the permeation resistance is large, the force for moving the particulate component towards the surface of the membrane becomes smaller than the force for detaching the particulate component from the surface of the membrane. Thus, the particulate component is not accumulated on the surface of the membrane.

The filtration membrane according to the present invention may comprise any of an inorganic material and an organic material. When it comprises an inorganic material, preferred examples of the inorganic compound include aluminum oxide, zirconium oxide, silicon oxide, tin oxide, titanium oxide, silicon carbide, silicon nitride and clay minerals. On the other hand, when it comprises an organic material, preferred examples of the organic compound include cellulose acetate, nitrocellulose, polyacrylonitrile, polyamide, polysulfone, polyethylene, polypropylene and polyethersulfone.

The filtration membrane according to the present invention may comprise a single layer of the above inorganic material or organic material. Alternatively, it may comprise a laminate of layers different from each other in the pore diameter or porosity. When the filtration membrane comprises a plurality of layers, it is needless to say that the construction of the membrane in such a manner that the whole membrane has the above average pore diameter or cut-off molecular weight and permeation resistance suffices for the present invention. The filtration membrane having a laminate structure is advantageous for the reason that, for example, when an increase in the thickness of the membrane for the purpose of imparting a strength to the membrane causes the filtration resistance to become excessively large, the strength can be imparted by reducing the thickness of a layer associated with the filtration (hereinafter often referred to as "active layer") and combining the thin layer with a layer having a large pore diameter.

Further, the filtration membrane according to the present invention may comprise a composite material composed of an inorganic material and an organic material. For example, it may comprise a filtration membrane composed of an inorganic material and, formed on the surface of the inorganic membrane, a thin membrane composed of an organic compound. The filtration membrane having such a structure has a combination of properties inherent in an inorganic membrane such as a high strength, an environmental resistance and a heat resistance with a fine screening capability inherent in an organic membrane. Although it is possible to impart a fine screening capability to an organic membrane, the organic membrane is inferior to the inorganic membrane in the strength, environmental resistance and heat resistance. In an organic thin membrane layer formed on the surface of an inorganic material, since the strength, environmental resistance and heat resistance are improved, the adoption of the above composite is advantageous.

According to a preferred embodiment of the present invention, the contact angle of the solution to be filtered with respect to the pore surface of the filtration membrane is 60° or less. When the contact angle of the solution to be filtered with respect to the surface of the filtration membrane is 60° or less, it is possible to prevent the adsorption of the component to be filtered on the filtration membrane. Since a macromolecular compound, such as a protein, adsorbed on pores of the filtration membrane is often detrimental to the permeability of the filtration membrane, it becomes possible to conduct the filtration with a higher efficiency through the prevention of the adsorption.

Further, according to another preferred embodiment, a sulfo group is present on the surface of pores of the filtration membrane. When a sulfo group is present on the surface of the filtration membrane, as with the case of the contact angle, the adsorption of the component to be filtered on the filtration membrane is prevented, so that an intended component can be recovered with a high efficiency.

According to a further preferred embodiment of the present invention, the filtration membrane is constructed so that the zeta potential of the surface of the membrane is zero or negative to that of the solution to be filtered. In this embodiment as well, as with the above embodiment, the adsorption of the component to be filtered on the filtration membrane is prevented, so that an intended component can be recovered with a high efficiency.

Filtration with Filtration Membrane

The filtration membrane according to the present invention is used in a configuration and a form suitable for the solution to be filtered and the filtration method. For example, when a cross flow filtration method is conducted, the filtration membrane is used as tubular form. Although the filtration conditions as well may be properly determined by taking into consideration the filtration method, for example, in the case of the cross flow filtration method, the filtration pressure and the flow rate of the surface of the membrane are preferably in the range from 0.5 to 2 kgf/cm$^2$ and in the range from about 1 to 5 m/sec, respectively.

Although there is no particular limitation on the application of the filtration by means of the filtration membrane according to the present invention, preferred examples of the application include a filtration operation wherein, in the conventional filtration membrane, an accumulated layer is unfavorably formed on the surface thereof, such as the recovery of an intended macromolecular compound, from a fermentation product of a microorganism.

In some cases, the permeated solution which has passed through the membrane contains other substances in addition to an intended component. Therefore, when it is desired to obtain an intended component in a more purified state, the purification may be conducted, for example, by chromatography or other conventional purification means.

Production of Filtration Membrane

The filtration membrane according to the present invention can be produced by a known method or a method similar thereto, except that the size of pores and the permeation resistance are set in respective predetermined ranges. A preferred example of the production of the filtration membrane according to the present invention will now be described.

When the filtration membrane comprises an inorganic material, the inorganic compound particles may be subjected to molding together with a binder and then sintered to give a porous material which may be used as a filtration membrane. The pore diameter or cut-off molecular weight can be determined by properly selecting the particle size of the inorganic compound as a starting material. The permeation resistance is inversely proportional to the second power of the pore diameter of the membrane, proportional to the membrane thickness and inversely proportional to the porosity (percentage of void). Therefore, a desired permeation resistance can be prepared through the regulation of the above three parameters. More specifically, the filtration membrane can be produced by a method described in Japanese Patent Application No. 418018/1990, Japanese Patent Laid-Open Publication No. 126924/1990, etc.

When the filtration membrane comprises an organic material, it may be produced according to a method for producing a conventional porous membrane. Also when the filtration membrane comprises an organic material, the permeation resistance is inversely proportional to the second power of the pore diameter of the membrane, proportional to the membrane thickness and inversely proportional to the porosity (percentage of void). Therefore, a filtration membrane having a desired permeation resistance can be prepared by properly selecting the pore diameter, membrane thickness and porosity of the membrane. More specifically, this filtration membrane may be produced according to a method described in, for example, Japanese Patent Laid-Open Publication Nos. 68410/1991 and 143535/1991, etc.

A membrane having a composite structure comprising an inorganic material and an organic material can be prepared, for example, by forming an organic compound layer on the surface of a filtration membrane comprising an inorganic material through the modification of the surface of the membrane with a silane coupling agent.

The contact angle of the solution to be filtered with respect to the filtration membrane can be reduced by introducing a hydrophilic functional group, for example, a hydroxyl group, a sulfo group or a carboxyl group, on the surface of the membrane. The contact angle can be increased by introducing a hydrophobic functional group, such as an alkyl group or a fluorine group, on the surface of the membrane. The introduction of various functional groups on the inorganic membrane can be conducted according to a method described in, for example, a paper by Shimizu et al. (Journal of the Ceramic Society of Japan, 95, 1067 (1987)). It is also possible to reduce the contact angle through the washing of the membrane with a strong acid, such as hydrochloric acid, nitric acid or sulfuric acid.

The introduction of a sulfo group on the pore surface of the filtration membrane and the regulation of the zeta potential of the surface of the filtration membrane to the solution to be filtered can be conducted by, besides the method described in the above paper by Shimizu et al., a method described in a paper by Shimizu et al. (Journal of Chemical Engineering of Japan, 22, 635 (1989)).

EXAMPLES

Example A1

(Production of Filtration Membrane)

A tubular internal pressure alumina filtration membrane (inner diameter: 7 mm, outer diameter: 10 mm, length: 200 mm) having an average pore diameter of 0.2 $\mu$m and a permeation resistance of $1 \times 10^{12}$ m$^{-1}$ was produced as follows.

Water was added to 92 parts by weight of an $\alpha$-alumina particle having an average particle diameter of 30 $\mu$m and 8 parts by weight of methyl cellulose as a binder, and they were mixed and extruded into a tube which was then dried and fired at 1750°C. A slurry comprising 95 parts by weight of an $\alpha$-alumina particle having a particle diameter of 0.5 $\mu$m, 5 parts by weight of methyl cellulose and water was flowed into the tube of the resultant porous material, and the water contained in a slurry was absorbed into the porous material to form a particulate layer on the surface of the porous material, thereby forming an active layer by the slip casting process. The water absorption time was 5 min. Thereafter, the resultant membrane was dried and then fired at 1200°C to form an alumina filtration membrane.

(Evaluation of Filtration Capability)

The filtration capability of the resultant membrane was evaluated as follows.

A cultivation product of strain Bacillus macerans IFO3940 which exports $\beta$-galactosidase into the extracellular milieu was prepared as a fermented solution to be filtered according to Miyazaki et al., Agric. Biol. Chem., 52, 625 (1988).

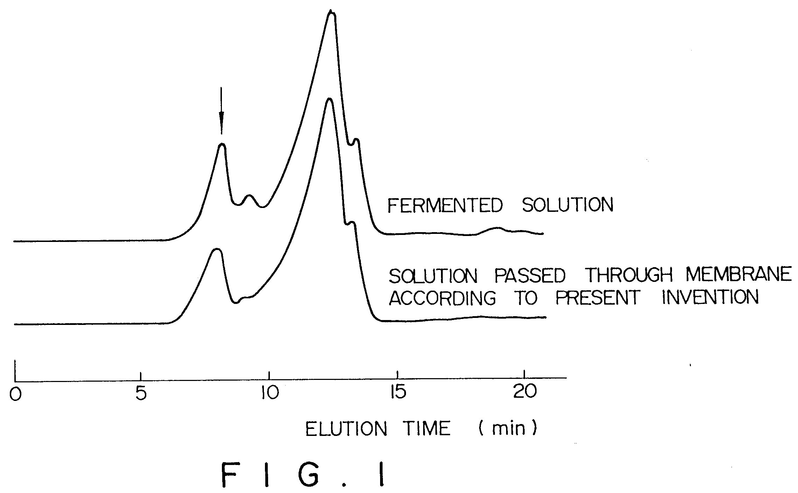

The filtration was conducted under cross flow conditions of a flow rate at a membrane surface of 1.0 m·s$^{-1}$ and a filtration pressure of 50 kPa. The $\beta$-galactosidase in the resultant permeated solution was analyzed by gel chromatography. The results are as shown in Fig. 1. Separately, the fermented solution, as such, was analyzed by gel chromatography. The results are also shown in Fig. 1.

In the gel chromatography, the conditions were set so that the retention time of the $\beta$-galactosidase was 8 min. When the results of analysis of the permeated solution was compared with those of the fermented solution, the peak ratio in the case of a retention time of 8 min was 0.75. This shows that the recovery of the $\beta$-galactosidase is 75%.

By a light scattering photometric analysis, it was confirmed that a particulate component was absent in the permeated solution. Therefore, it can be judged that no particulate component leaked into the permeated solution.

Further, the presence or absence of the accumulated layer was estimated as follows. The membrane permeation flux value was measured before and after the filtration. The membrane permeation flux value before the filtration and that after the filtration were designated as PWF and J, respectively. Further, the filtration membrane after the completion of the filtration was washed with a sponge and subjected to measurement of the membrane permeation flux value, PWF', under conditions of a temperature of 30°C and a pressure of 50 kPa. The results were as follows.

PWF = 5.31 m$^3$·m$^{-2}$d$^{-1}$
J = 4.11 m$^3$·m$^{-2}$d$^{-1}$
PWF' = 4.15 m$^3$·m$^{-3}$d$^{-1}$

When the permeation resistance, the increase in the permeation resistance by clogging of the membrane and the resistance of the accumulated layer are Rm, Rp and Rc, respectively, the following relationship is established.

$$1/PWF = (\Delta P/\eta)·Rm$$
$$1/J = (\Delta P/\eta)·(Rm + Rp + Rc)$$
$$1/PWF' = (\Delta P/\eta)·(Rm + Rp)$$

The Rm, Rp and Rc values were determined according to the above equations, and the results were as follows.

Rm × 10$^{-11}$ (m$^{-1}$) 10.0
Rp × 10$^{-11}$ (m$^{-1}$) 2.8
Rc × 10$^{-11}$ (m$^{-1}$) 0.1

Thus, it is apparent that in the present Example, substantially no accumulated layer was formed on the surface of the filtration membrane.

Example A2

An alumina asymmetric membrane having a permeation resistance of 6 × 10$^{11}$ m$^{-1}$ was produced in the same manner as that of Example A1, except that the water absorption time of slurry was 3 min. A fermented solution of a microorganism was filtered in the same manner as that of Example A1 through the resultant membrane.

The fermented solution and the solution passed through the membrane were subjected to gel chromatography. The recovery of the $\beta$-galactosidase determined from the peak ratio of the permeated solution to the fermented solution was 68%.

The Rm, Rp and Rc values were determined in the same manner as that of Example A1 and found to be as follows.

Rm × 10$^{-11}$ (m$^{-1}$) 6.0
Rp × 10$^{-11}$ (m$^{-1}$) 1.2
Rc × 10$^{-11}$ (m$^{-1}$) 0.2

Thus, it is apparent that in the present Example, substantially no accumulated layer was formed on the surface of the filtration membrane.

Example A3

(Production of Filtration Membrane)

A filtration membrane having an average pore diameter of 10 nm and a permeation resistance of 11 × 10$^{11}$ m$^{-1}$ was produced as follows. A tube of a porous material produced in Example A1 was provided, and a slurry comprising 95 parts by weight of an $\alpha$-alumina particle having an average particle diameter of 20 nm and 5 parts by weight of methyl cellulose was flowed into this tube. The water absorption time was 1 min. Then, the resultant membrane was dried and fired at 1000°C to form a filtration membrane.

(Evaluation of Filtration Capability)

The collection of an intended substance from a disrupted product of cells by means of the membrane prepared above was conducted as follows.

The solution to be filtered was prepared as follows. Escherichia coli was subjected to cultivation in a complete medium, and the cultivation product was subjected to centrifugation to collect cells which were washed with physiological saline and re-dispersed in physiological saline. The dispersion was disrupted by means of a French press, and bovine serum albumin was added to the disrupted dispersion to prepare a model solution.

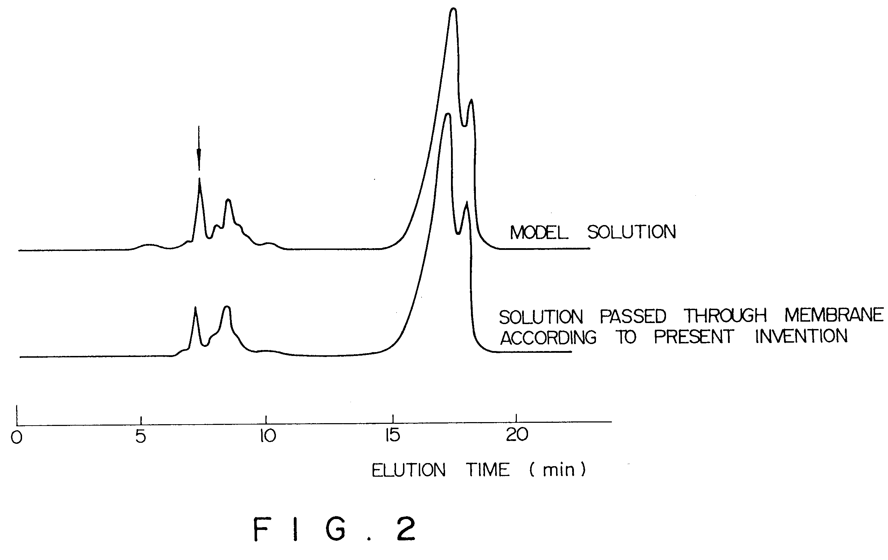

The filtration was conducted under cross flow conditions of a flow rate of 1.0 m•s$^{-1}$ and a filtration pressure of 50 kPa. The bovine serum albumin in the permeated solution was analyzed by gel chromatography. The results are as shown in Fig. 2. Separately, the model solution, as such, was analyzed by gel chromatography. The results are also as shown in Fig. 2.

In the gel chromatography, the conditions were set so that the retention time of the $\beta$-galactosidase was 7.2 min. When the results of analysis of the permeated solution was compared with those of the model solution, the recovery of the bovine serum albumin was 72%.

Example B1

(Production of Filtration Membrane)

A tubular internal pressure alumina filtration membrane (inner diameter: 7 mm, outer diameter: 10 mm, length: 200 mm) having an average pore diameter of 0.2 $\mu$m, a permeation resistance of 6 × 10$^{11}$ m$^{-1}$ and a contact angle of purified water of 30° were produced as follows.

The filtration membrane prepared in Example A2 was refluxed in a toluene solution containing 5 parts by weight of trimethylchlorosilane for 20 hr, washed with toluene, dried and fired at 600°C to prepare a filtration membrane having a monomolecular layer of silica formed on the surface thereof.

(Evaluation of Filtration Capability)

The filtration capability of the membrane prepared above was evaluated in the same manner as that of Example A1.

As a result of gel chromatography, it was found that the recovery of the $\beta$-galactosidase was 85%. By a light scattering photometric analysis, it was confirmed that a particulate component was absent in the permeated solution.

The Rm, Rp and Rc values were determined in the same manner as that of Example A1. The results were as follows.
Rm × 10$^{-11}$ (m$^{-1}$) 6.0
Rp × 10$^{-11}$ (m$^{-1}$) 0.8
Rc × 10$^{-11}$ (m$^{-1}$) 0.1

Since the Rp value is small, it is estimated that substantially no accumulated layer of the particulate component is formed. Further, since the Rc value is also small, it is believed that the adsorption to the filtration membrane is suppressed on a low level.

Example B2

(Production of Filtration Membrane)

An asymmetric zirconia filtration membrane having an average pore diameter of 10 nm, a permeation resistance of 11 × 10$^{11}$ m$^{-1}$ and a contact angle of a purified water of 42° was produced in the same manner as that of Example A3, except that a zirconia particle having an average particle diameter of 20 nm was used instead of $\alpha$-alumina.

(Evaluation of Filtration Capability)

The filtration capability of the membrane prepared above was evaluated in the same manner as that of Example A3.

As a result, it was found that the bovine serum albumin could be recovered from the model solution with an efficiency of 72%.

Example C1

(Production of Filtration Membrane)

A tubular internal pressure alumina filtration membrane having an average pore diameter of 0.2 $\mu$m, a permeation resistance of 6 × 10$^{11}$ m$^{-1}$ and a sulfo group introduced on the surface thereof was produced as follows.

A sulfo group was introduced in 4 molecules/nm$^2$ on the surface of pores in the tubular internal pressure alumina filtration membrane produced in Example A1. The introduction of the sulfo group was conducted by refluxing the alumina filtration membrane in a solution of dimethylphenylchlorosilane in toluene for 5 hr to replace a hydroxyl group on the surface of alumina with a phenyl group and then treating the membrane with 4.5 N sulfuric acid for 2 hr to convert the phenyl group to a sulfophenyl group.

(Evaluation of Filtration Capability)

The filtration capability of the membrane prepared above was evaluated in the same manner as that of Example A1.

As a result of gel chromatography, it was found that the recovery of the $\beta$-galactosidase was 90%. By a light scattering photometric analysis, it was confirmed that a particulate component was absent in the permeated solution.

The Rm, Rp and Rc values were determined in the same manner as that of Example A1. The results were as follows.
Rm × 10$^{-11}$ (m$^{-1}$) 6.0
Rp × 10$^{-11}$ (m$^{-1}$) 0.2
Rc × 10$^{-11}$ (m$^{-1}$) 0.1

Since the Rp value is small, it is estimated that substantially no accumulated layer of the particulate component is formed. Further, since the Rc value is also small, it is also believed that the adsorption to the filtration membrane is suppressed on a low level.

Example C2

A filtration membrane having a pore diameter of 10 nm and a permeation resistance of $11 \times 10^{11}$ $m^{-1}$ was prepared by introducing a sulfo group into the filtration membrane produced in Example A3 in the same manner as that of Example C1.

The filtration performance of the filtration membrane was evaluated in the same manner as that of Example A3. As a result, it was found that the bovine serum albumin could be recovered from the model solution with an efficiency of 85%.

Example D1

(Production of Filtration Membrane)

A tubular internal pressure alumina filtration membrane having an average pore diameter of 0.2 $\mu$m, a permeation resistance of $6 \times 10^{11}$ $m^{-1}$ and a zeta potential of -10 mV to the fermented solution prepared in Example A1 was produced by immersing the tubular internal pressure alumina filtration membrane produced in Example A1 in 5 N sulfuric acid or 90°C for 1 hr.

(Evaluation of Filtration Capability)

The filtration capability of the membrane prepared above was evaluated in the same manner as that of Example A1.

As a result of gel chromatography, it was found that the recovery of the $\beta$-galactosidase was 85%. By a light scattering photometric analysis, it was confirmed that a particulate component was absent in the permeated solution.

The Rm, Rp and Rc values were determined in the same manner as that of Example A1. The results were as follows.
Rm $\times 10^{-11}$ ($m^{-1}$) 6.0
Rp $\times 10^{-11}$ ($m^{-1}$) 0.8
Rc $\times 10^{-11}$ ($m^{-1}$) 0.1
Since the Rp value is small, it is estimated that substantially no accumulated layer of the particulate component is formed. Further, since the Rc value is also small, it is believed that the adsorption to the filtration membrane is suppressed on a low level.

Example D2

A filtration membrane having an average pore diameter of 10 nm, a permeation resistance of $11 \times 10^{11}$ $m^{-1}$ and a zeta potential of 0 mV to the fermented solution prepared in Example A1 was produced by subjecting the surface of the filtration membrane produced in Example A3 to the following treatment.

The filtration membrane prepared in Example A1 was refluxed in a solution of dimethylphenylchlorosilane in toluene for 5 hr to replace a hydroxyl group present on the surface of alumina with a phenyl group and immersing the filtration membrane in 1 N sulfuric acid for 10 min to partially convert the phenyl group to a sulfophenyl group to give a filtration membrane.

The filtration performance of the filtration membrane was evaluated in the same manner as that of Example A3. As a result, it was found that the bovine serum albumin could be recovered from the model solution with an efficiency of 75%.

Comparative Example 1

The separation of $\beta$-galactosidase was conducted in the same manner as that of Example A1, except that a commercially available alumina asymmetric membrane (alumina micro filtration membrane T-0.2M manufactured by Toshiba Ceramics Co., Ltd. and having an average pore diameter of 0.2 $\mu$m, a permeation resistance of $1 \times 10^{11}$ $m^{-1}$, a contact angle of pure water of 30°, a surface functional group, such as a hydroxyl group (but not a sulfo group), and a zeta potential of 0 mV to the fermented solution prepared in Example A1) was used as the filtration membrane.

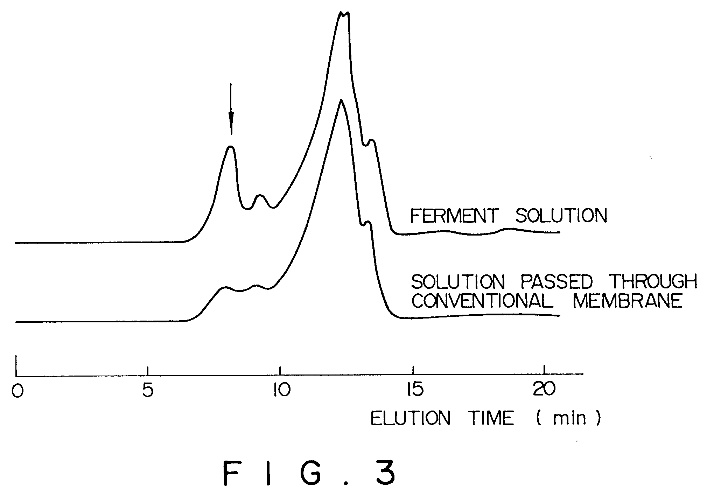

The fermented solution and the solution passed through the membrane were subjected to gel chromatography. The results are as shown in Fig. 3. The recovery of the $\beta$-galactosidase determined from the peak ratio of the permeated solution to the fermented solution was 20%.

The Rm, Rp and Rc values were determined in the same manner as that of Example A1. The results were as follows.
Rm $\times 10^{-11}$ ($m^{-1}$) 1.0
Rp $\times 10^{-11}$ ($m^{-1}$) 0.8
Rc $\times 10^{-11}$ ($m^{-1}$) 4.2
Thus, it is apparent that, in this filtration membrane, an accumulated layer is unfavorably formed on the surface of the filtration membrane.

Comparative Example 2

The separation of bovine serum albumin was conducted in the same manner as that of Example A3, except that a commercially available ultrafiltration membrane (PC cassette having a cut-off molecular weight of 50,000 manufactured by Mitsui Petrochemical Industries, Ltd.) was used as the filtration membrane. As a result, the recovery of the bovine serum albumin in the permeated solution was 10% or less.

## Claims

1. A filtration membrane with pores having an average pore diameter in the range from 10 nm to 10 $\mu$m or pores defined by a cut-off molecular weight in the range from $1 \times 10^4$ to $1 \times 10^8$, said membrane having a permeation resistance in the range from $5 \times 10^{11}$ m$^{-1}$ to $1 \times 10^{15}$ m$^{-1}$.

2. A filtration membrane according to claim 1, wherein a contact angle of a solution to be filtered with respect to the membrane is 60° or less.

3. A filtration membrane according to claim 1, wherein the membrane has a sulfo group on the surface thereof.

4. A filtration membrane according to claim 1, wherein the zeta potential of the membrane to a solution to be filtered is zero or negative.

5. A filtration membrane according to any one of claim 1 to 4, which comprises an inorganic material.

FIG. 1

FERMENTED SOLUTION

SOLUTION PASSED THROUGH MEMBRANE
ACCORDING TO PRESENT INVENTION

ELUTION TIME (min)

EP 0 503 087 A1

FIG. 2

FERMENT SOLUTION

SOLUTION PASSED THROUGH
CONVENTIONAL MEMBRANE

ELUTION TIME ( min )

F I G . 3

EP 0 503 087 A1

# INTERNATIONAL SEARCH REPORT

International Application No PCT/JP91/01311

## I. CLASSIFICATION OF SUBJECT MATTER (if several classification symbols apply, indicate all) 6

According to International Patent Classification (IPC) or to both National Classification and IPC

Int. Cl$^5$ B01D69/02, 71/02

## II. FIELDS SEARCHED

### Minimum Documentation Searched 7

| Classification System | Classification Symbols |
|---|---|
| IPC | B01D69/00, 69/02, 71/02, 71/82 |

### Documentation Searched other than Minimum Documentation to the Extent that such Documents are Included in the Fields Searched 8

| | |
|---|---|
| Jitsuyo Shinan Koho | 1926 - 1990 |
| Kokai Jitsuyo Shinan Koho | 1971 - 1990 |

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| X | JP, A, 2-63514 (Terumo Corp.), March 2, 1990 (02. 03. 90), Line 12 to 2nd line from the bottom, lower left column, page 2, Tables 1, 2 (Family: none) | 1, 2, 5 |
| Y | JP, A, 2-63514 (Terumo Corp.), March 2, 1990 (02. 03. 90), Line 12 to 2nd line from the bottom, lower left column, page 2 (Family: none) | 3-4 |
| Y | JP, A, 2-133448 (Asahi Glass Co., Ltd.), May 22, 1990 (22. 05. 90), Line 10, upper left column to 3rd line from the bottom, upper right column, page·3 (Family: none) | 1-5 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| November 29, 1991 (29. 11. 91) | December 17, 1991 (17. 12. 91) |
| International Searching Authority | Signature of Authorized Officer |
| Japanese Patent Office . | |

Form PCT/ISA/210 (second sheet) (January 1985)